(19) Europäisches Patentamt
European Patent Office
Office européen des brevets

(11) Veröffentlichungsnummer : **0 473 021 B1**

(12) # EUROPÄISCHE PATENTSCHRIFT

(45) Veröffentlichungstag der Patentschrift : **04.05.94 Patentblatt 94/18**

(51) Int. Cl.[5] : **C07D 309/22,** C08L 23/00

(21) Anmeldenummer : **91113713.1**

(22) Anmeldetag : **16.08.91**

(54) **2-(3'-Butenyl)-3,4-dihydro-2H-pyrane, ihre Herstellung und Verwendung.**

(30) Priorität : **30.08.90 DE 4027436**

(43) Veröffentlichungstag der Anmeldung : **04.03.92 Patentblatt 92/10**

(45) Bekanntmachung des Hinweises auf die Patenterteilung : **04.05.94 Patentblatt 94/18**

(84) Benannte Vertragsstaaten : **BE DE FR GB IT NL**

(56) Entgegenhaltungen : **CHEMICAL ABSTRACTS, Band 109, Nr. 17, 24. Oktober 1988, Columbus, Ohio, USASINGH, S.M. et al. "Chelation vs. non-chelation control in addition reactionsof ethylmetallic reagnets to acrolein dimer" Seite 711, Spalte 1, Zu-sammenfassung-Nr. 149 286z**

(56) Entgegenhaltungen : **CHEMICAL ABSTRACTS, Band 103, Nr. 13, 30. September 1985, Columbus, Ohio, USABHUPATHY, M. et al. "Remarkable stereocontrol in the addition of an anion to analpha-alkoxy-aldehyde by encouraging or discouraging internal complexation.Applications to brief syntheses of the Mus musculus (house mouse) pheromone and exo-brevicomin" Seite 600, Spalte 1, Zusammenfassung-Nr. 104 746j**

(73) Patentinhaber : **Th. Goldschmidt AG**
**Goldschmidtstrasse 100**
**D-45127 Essen (DE)**

(72) Erfinder : **Schaefer, Dietmar, Dr.**
**Im Poth 12**
**W-4320 Hattingen 16 (DE)**
Erfinder : **Weier, Andreas, Dr.**
**Am Wasserturm 36**
**W-4300 Essen 1 (DE)**
Erfinder : **Weitemeyer, Christian, Dr.**
**Sundernholz 67**
**W-4300 Essen 1 (DE)**
Erfinder : **Wewers, Dietmar, Dr.**
**Am Köllnischen Wald 6**
**W-4250 Bottrop (DE)**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 473 021 B1

## Beschreibung

Die Erfindung betrifft neue, kationisch härtbare 2-(3'-Butenyl)-3,4-dihydro-2H-pyrane, ihre Herstellung und Verwendung, insbesondere als kationisch härtbare Vergußmassen oder Beschichtungsmittel für flächige Träger oder als reaktive Modifizierungs- oder Verdünnungsmittel für kationisch härtbare Monomere oder Polymerisate.

Neben den bei UV-Einwirkung härtenden radikalisch polymerisierenden Systemen sind in den letzten Jahren kationisch härtbare, Vinylverbindungen enthaltende Systeme entwickelt worden, bei denen die Härtung insbesondere durch Diaryliodonium- und Triarylsulfoniumsalze initiiert wird. Der Vorteil der kationisch härtenden Systeme liegt in der Unempfindlichkeit der Härtungsreaktion gegen Luftsauerstoff, die rasche Filmbildung und die Umweltfreundlichkeit dieser Systeme.

Die kationische Härtung von Vinylmonomeren ist in den US-Patentschriften 4 617 238, 4 518 788 und 4 705 887 beschrieben.

Dabei haben die UV-härtbaren Vinyletherverbindungen aufgrund ihrer schnellen Härtbarkeit, der wirtschaftlichen Verarbeitbarkeit und ihrer Umweltfreundlichkeit besondere Beachtung gefunden. Derartige Vinyletherverbindungen lassen sich auf verschiedene Weise herstellen. In einem Übersichtsreferat über "Geschwindigkeitsbestimmende Faktoren bei der kationischen UV-Härtung" in der Zeitschrift Farbe und Lack, 1987, Seiten 803 bis 807, werden folgende Synthesemöglichkeiten angegeben:

1. Basenkatalysierte Acetylenaddition an Diole nach Reppe

$$HO\text{-}R\text{-}OH + 2\ HC{\equiv}CH \xrightarrow{NaOH} R\text{-}(O\text{-}CH{=}CH_2)_2$$

2. Phasentransferkatalysierte Kondensation von 2-Chlorethylvinylether und Diolen

$$HO\text{-}R\text{-}OH + 2\ Cl\text{-}CH_2\text{-}CH_2O\text{-}CH{=}CH_2 \xrightarrow[(C_4H_9)_4N^+Br^-]{NaOH} R\text{-}(O\text{-}CH_2\text{-}CH_2\text{-}O\text{-}CH{=}CH_2)_2$$

3. Katalysierte Umlagerung der Bis-allylether in die entsprechenden Bis-methylvinylether

$$R\text{-}(O\text{-}CH_2\text{-}CH{=}CH_2)_2 \xrightarrow{[(C_6H_5)_3P]_3RuCl_2} R\text{-}(O\text{-}CH{=}CH\text{-}CH_3)_2$$

Die Addition nach Reppe ist unter wirtschaftlichen Gesichtspunkten nicht realisierbar. Bei der zweiten Reaktion benötigt man 2-Chlorethylether, dessen Einsatz aus physiologischen Gründen unerwünscht ist.

Den Vinylethern ist der Nachteil gemeinsam, daß in Gegenwart von Feuchtigkeit und Spuren Säure eine Aufspaltung der Enol-Ether in niedrigsiedende, geruchsintensive Carbonylverbindungen erfolgt:

$$R\text{-}O\text{-}CR^*{=}CR^*_2 + H_2O \xrightarrow{H^+} R\text{-}O\text{-}H + O{=}CR^*\text{-}CR^*_3$$

R* = H- oder Alkylrest

Mit der vorliegenden Erfindung soll das technische Problem der Bereitstellung weiterer kationisch härtbarer Verbindungen gelöst werden, die selbst oder als reaktive Modifizierungs- oder Verdünnungsmittel für kationisch härtbare Monomere oder Polymerisate eingesetzt werden können, und unter vergleichbaren Bedingungen keine niedrigsiedenden, geruchsintensiven Carbonylverbindungen freisetzen. Dabei sollen die kationisch härtbaren Verbindungen in möglichst einfacher und wirtschaftlicher Weise hergestellt werden können.

Die erfindungsgemäßen neuen kationisch härtbaren Verbindungen entsprechen der allgemeinen Formel

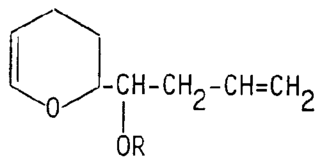

I

wobei R ein Wasserstoff- oder Alkylrest mit 1 bis 6 Kohlenstoffatomen ist.

Beispiele geeigneter Alkylreste R sind der Methyl-, Ethyl-, Propyl-, iso-Propyl-, Butyl-, iso-Butylrest, Pentyl- und Hexylrest. Bevorzugt sind die geradkettigen Alkylreste, insbesondere der Ethylund der Propylrest.

Besonders bevorzugte Verbindungen sind die nachstehend aufgeführten Verbindungen:

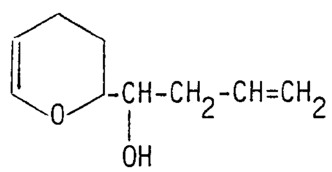

,

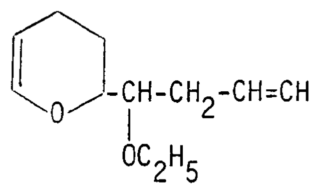

,

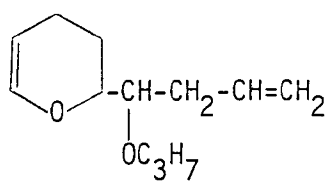

Ein weiterer Gegenstand der vorliegenden Erfindung besteht in der Herstellung der erfindungsgemäßen neuen Verbindungen. Das erfindungsgemäße Verfahren ist dadurch gekennzeichnet, daß man entweder

a) 2-Formyldihydropyran mit Allylmagnesiumhalogenid im Molverhältnis 1 : 0,8 bis 1 : 1,3 in Gegenwart eines Ethers oder Ether/Alkan-Gemisches als Lösungsmittel innerhalb eines Temperaturbereiches von -80 bis +80°C umsetzt, oder

b) 2-Formyldihydropyran mit Allyllithium im Molverhältnis 1 : 1 bis 1 : 1,3 in Gegenwart eines Ethers oder Ether/Alkan-Gemisches als Lösungsmittel innerhalb eines Temperaturbereiches von -80 bis +70°C umsetzt, und

c) das nach a) oder b) erhaltene Reaktionsprodukt hydrolysiert und anschließend, falls $R^2$ ein Alkylrest ist, in an sich bekannter Weise mit einem Halogenid der Formel $R^2X$ (X = Halogen) verethert.

Das Verfahren a) verläuft als Grignard-Reaktion in an sich bekannter Weise. Als Lösungsmittel können die hierfür bekannten Lösungsmittel verwendet werden. Dies sind in erster Linie Ether, wie Tetrahydrofuran oder Diethylether oder die Gemische dieser Ether mit Alkanen, wie Hexan. Der bevorzugte Temperaturbereich liegt bei 30 bis 70°C. Zur Hydrolyse empfiehlt es sich, das Reaktionsgemisch in Eiswasser einzugießen. Das Reaktionsgemisch wird als ölige Phase vom Wasser abgetrennt und kann dann in üblicher Weise, z.B. durch Destillation, gereinigt werden.

Das Verfahren b) läuft ebenfalls in Gegenwart von Ethern oder Ether/Alkan-Gemischen als Lösungsmittel ab. Der bevorzugte Temperaturbereich ist -80 bis +20°C.

Die Veretherung in Schritt c) erfolgt in ebenfalls bekannter Weise durch Umsetzung der Reaktionsprodukte mit Alkylhalogeniden bei etwa 20 bis 100°C.

Die erfindungsgemäßen Verbindungen sind niedrigviskose, wasserklare Flüssigkeiten.

Ein weiterer Gegenstand der Erfindung besteht in der Verwendung der erfindungsgemäßen Substanzen als kationisch härtbare Vergußmassen oder Beschichtungsmittel für flächige Träger oder als reaktive Modifizierungsmittel für kationisch härtbare Monomere oder Polymerisate.

Die Aushärtung der erfindungsgemäßen Verbindungen erfolgt vorzugsweise durch UV-Strahlen in Gegenwart von Katalysatoren, wie salzartigen Diaryliodonium- oder Triarylsulfoniumverbindungen oder nichtsalzartigen Verbindungen, wie Ketosulfonen. Beispiele solcher Härter sind

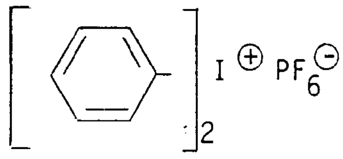 ; 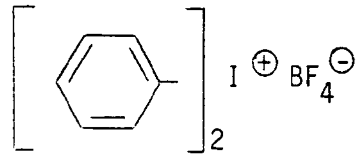

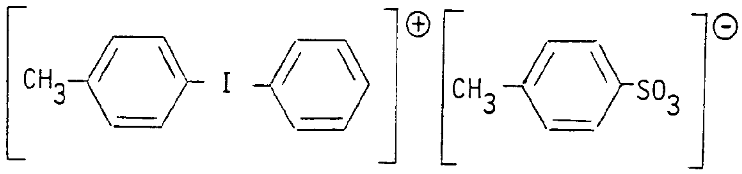

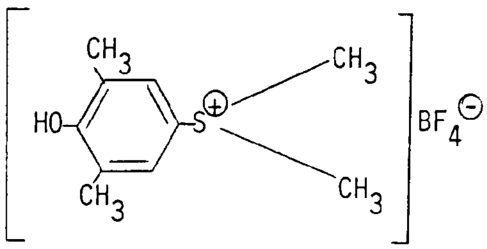 ; 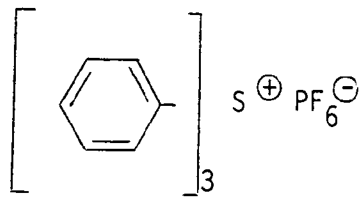

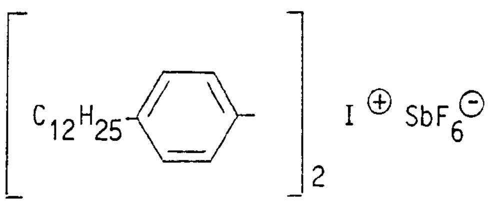 ; 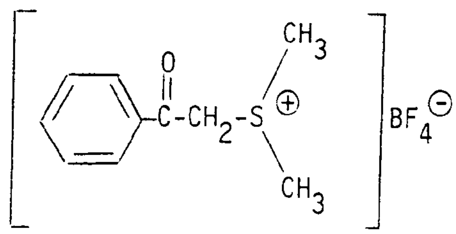

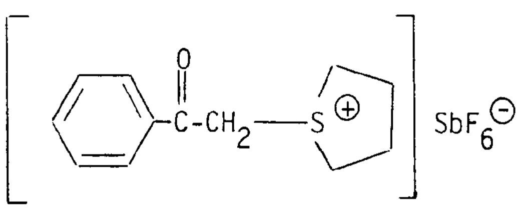 ; 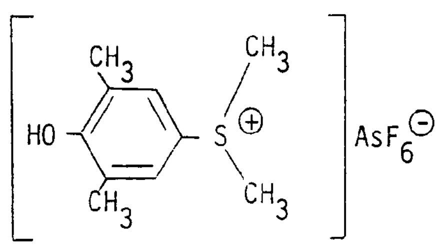

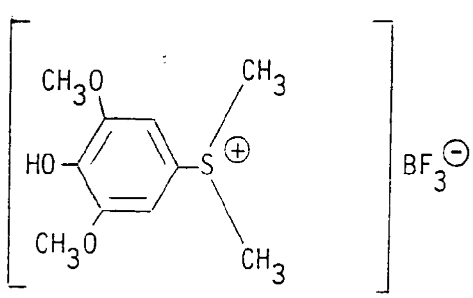

Besonders bevorzugte Initiatoren sind:

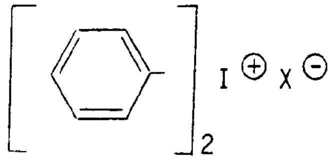 ; 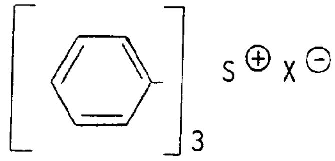

$X^{\ominus} = PF_6^{\ominus}$ ; $BF_4^{\ominus}$ ; $SbF_6^{\ominus}$

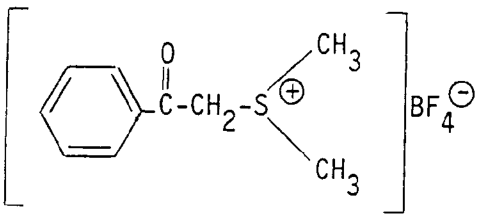 ; 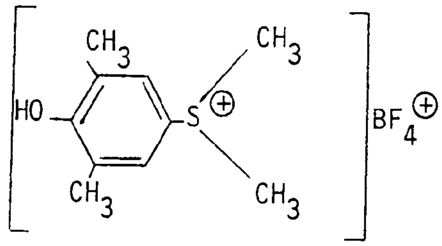

Des weiteren ist eine Hitzehärtung unter Verwendung von Oniumsalzen mit organischen Oxidationsmitteln oder löslichen Kupfersalzen oder Chelaten möglich, wie sie z. B. in der US-PS 4 173 551 beschrieben ist. Eine andere Möglichkeit, die Härtung herbeizuführen, besteht in der Verwendung von Verbindungen, die bei Temperaturerhöhung Säuren oder Lewissäuren freisetzen, wie Sulfonsäuresalze, besonders Aminsalze, Sulfonsäureester und Aminkomplexe von Lewissäuren, wie z.B. der Borsäuretrifluoridtriethylaminkomplex.

Die Härter werden den erfindungsgemäßen Verbindungen in Mengen von etwa 0,1 bis 5 Gew.-% zugesetzt.

Die erfindungsgemäßen Verbindungen können mit üblichen Zusatzmitteln, wie Füllstoffen, Pigmenten, Flammschutzmitteln und dergleichen vermischt werden.

Die härtbaren Verbindungen oder deren Zubereitungen können auf Substrate wie Metall, Gummi, Kunststoff, Papier, Holz, Glasgewebe, Zement, Keramik usw. aufgebracht werden.

Die erfindungsgemäßen Verbindungen können Polymerisaten zugemischt werden, die entweder selbst kationisch härtbar sind oder die reaktive Gruppen aufweisen, die mit der endständigen Doppelbindung der erfindungsgemäßen Verbindungen reagieren können. Beispiele solcher Polymerisate sind Propenylpolyether, Vinylpolyetherpolysiloxane, Epoxygruppen enthaltende kohlenstofforganische oder siliciumorganische Verbindungen, Styrol, Wasserstoffsiloxane (bei Anwesenheit von Übergangsmetallkatalysatoren).

In den folgenden Beispielen werden die Herstellung der erfindungsgemäßen Verbindungen und deren anwendungstechnische Eigenschaften noch näher und erläuternd gezeigt.

## Beispiel 1

Zu 48 g (2,0 Mol) Magnesiumspänen in 400 ml eines 5 : 3 Gemisches von wasserfreiem THF und Diethylether werden eine Mischung von 242 g (2,0 Mol) Allylbromid und 179,2 g 2-Formyldihydropyran langsam zugetropft, so daß die Reaktionsmischung kontinuierlich siedet. Nach Ende des Zutropfens wird noch 5 h am Rückfluß gekocht. Danach wird die Reaktionsmischung auf 1 l Eiswasser gegeben, die organische Phase abgetrennt und die wäßrige Phase mit 200 ml gesättigter Ammoniumchlorid-Lösung verdünnt und mit 200 ml Hexan gewaschen. Die vereinigten organischen Phasen werden über Magnesiumsulfat getrocknet, filtriert und destilliert. Man erhält 129 g (52 % der Theorie) einer farblosen Flüssigkeit vom Siedepunkt 85°C / 2,7 mbar, bei der es sich laut NMR-spektroskopischer Untersuchung um 2-(1'-Hydroxy-3'-butenyl)-3,4-dihydro-2H-pyran handelt.

## Beispiel 2

Eine Suspension von 22,56 g (0,4 Mol) gepulvertem KOH in 100 ml Dimethylsulfoxid (DMSO) wird 5 min gerührt, dann werden schnell hintereinander 15,42 g (0,1 Mol) 2-(1'-Hydroxy-3'-butenyl)-3,4-dihydro-2H-pyran und 21,8 g (0,2 Mol) Ethylbromid zugegeben. Nach 30 min Rühren wird das Reaktionsgemisch in 500 ml Wasser gegossen und dreimal mit je 50 ml Diethylether extrahiert. Die vereinigten organischen Phasen werden fünfmal mit je 30 ml Wasser gewaschen, über Magnesiumsulfat getrocknet und destilliert. Man erhält 15,6 g (86 % der Theorie) des erwarteten Ethylethers.

Beispiel 3

Analog zu Beispiel 2 werden aus 22,56 g (0,4 Mol) gepulvertem KOH, 15,42 g (0,1 Mol) 2-(1'-Hydroxy-3'-butenyl)-3,4-dihydro-2H-pyran und 24,6 g (0,2 Mol) Propylbromid 16,3 g (83 % der Theorie) 2-(1'-Propoxy-3'-butenyl)-3,4-dihydro-2H-pyran erhalten.

Zur Überprüfung der Verwendbarkeit der erfindungsgemäßen Dihydropyrane als reaktive Verdünner werden die Substanzen anderen kationisch härtenden Substanzen (einer Mischung von Bisphenol-A-diglycidether und Triethylenglycoldivinylether im Verhältnis 1 : 1, im folgenden "BTE" genannt) zugesetzt und nach Zugabe von 2 Gew.-% eines geeigneten Photoinitiators durch UV-Bestrahlung (Mitteldruck-Quecksilberdampflampe, 80 W/cm) in einem Aluminiumdeckel (Durchmesser 50 mm) gehärtet. Die Schichtdicke beträgt in jedem Fall etwa 3 mm.

| Substanz | Viskosität | Härtung | Oberfläche |
|---|---|---|---|
| BTE | 10 cp | 10 s | fest, schmierfrei, brüchig |
| BTE + 5 % Bsp. 1 | 8,0 cp | 9 - 10 s | fest, schmierfrei, unelastisch |
| BTE +10 % Bsp. 1 | 7,6 cp | 9 - 10 s | fest, schmierfrei, unelastisch |
| BTE + 5 % Bsp. 2 | 8,6 cp | 10 s | fest, schmierfrei, elastisch |
| BTE + 5 % Bsp. 3 | 9,0 cp | 10 s | fest, schmierfrei, elastisch |

Die Substanzen härten nach UV-Bestrahlung, ohne durch Luftsauerstoff inhibiert zu werden.

Dabei sind besonders die Alkohole durch ihre relativ aciden Protonen befähigt, die Härtungsgeschwindigkeit zu erhöhen. Die Oberflächenbeschaffenheit ändert sich bei Zugabe der Dihydropyrane, so daß die ausgehärteten Substanzen weniger spröde sind.

## Patentansprüche

**1.** 2-(3'-Butenyl)-3,4-dihydro-2H-pyrane der allgemeinen Formel

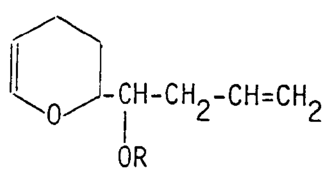

I

wobei R ein Wasserstoff- oder Alkylrest mit 1 bis 6 Kohlenstoffatomen ist.

**2.** Verfahren zur Herstellung von Verbindungen der Formel I, dadurch gekennzeichnet, daß man entweder

a) 2-Formyldihydropyran mit Allylmagnesiumhalogenid im Molverhältnis 1 : 0,8 bis 1 : 1,3 in Gegenwart eines Ethers oder Ether/Alkan-Gemisches als Lösungsmittel innerhalb eines Temperaturbereiches von -80 bis +80°C umsetzt, oder

b) 2-Formyldihydropyran mit Allyllithium im Molverhältnis 1 : 0,8 bis 1 : 1,3 in Gegenwart eines Ethers oder Ether/Alkan-Gemisches als Lösungsmittel innerhalb eines Temperaturbereiches von -80 bis +70°C umsetzt, und

c) das nach a) oder b) erhaltene Reaktionsprodukt hydrolysiert und anschließend, falls $R^2$ ein Alkylrest ist, in an sich bekannter Weise mit einem Halogenid der Formel $R^2X$ (X = Halogen) verethert.

**3.** Verfahren nach Anspruch 2, dadurch gekennzeichnet, daß die Umsetzung bei der Verfahrensweise a) bei 30 bis 70°C und bei der Verfahrensvariante b) bei -80 bis +20°C durchgeführt wird.

**4.** Verwendung der Verbindungen nach Anspruch 1 als kationisch härtbare Vergußmassen oder Beschichtungsmittel für flächige Träger oder als reaktive Modifizierungs- oder Verdünnungsmittel für kationisch härtbare Monomere oder Polymerisate.

## Claims

1. 2-(3'-Butenyl)-3,4-dihydro-2H-pyrans of the general formula

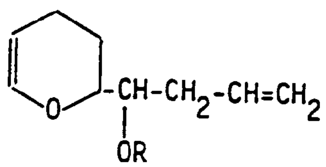

I

wherein R is a hydrogen radical or an alkyl radical having 1 to 6 carbon atoms.

2. Process for the preparation of compounds of the formula I, characterized in that either
   a) 2-formyldihydropyran is reacted with allylmagnesium halide in a molar ratio of 1:0.8 to 1:1.3 in the presence of an ether or ether/alkane mixture as the solvent within a temperature range of -80 to +80°C, or
   b) 2-formyldihydropyran is reacted with allyllithium in a molar ratio of 1:0.8 to 1:1.3 in the presence of an ether or ether/alkane mixture as the solvent within a temperature range of -80 to +70°C, and
   c) the reaction product obtained by a) or b) is hydrolysed and subsequently, if $R^2$ is an alkyl radical, etherified with a halide of the formula $R^2X$ (X=halogen) in a manner which is known per se.

3. Process according to Claim 2, characterized in that the reaction is carried out at 30 to 70°C in the case of procedure a) and at -80 to +20°C in the case of process variant b).

4. Use of the compounds according to Claim 1 as cationically curable casting compositions or coating agents for sheet-like supports or as reactive modifying agents or diluents for cationically curable monomers or polymers.

## Revendications

1. 2-(3'-butényl)-3,4-dihydro-2H-pyrannes de formule générale

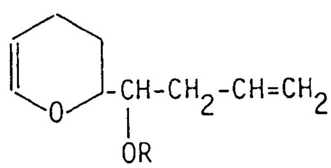

I

où R représente un reste d'hydrogène ou un reste alkyle ayant de 1 à 6 atomes de carbone.

2. Procédé de préparation des composés de formule I, caractérisé en ce que
   a) on fait réagir du 2-formyldihydropyranne avec de l'halogénure d'allylmagnésium, selon un rapport molaire valant de 1 : 0,8 à 1 : 1,3, en présence d'un éther ou d'un mélange d'éther et d'alcane, comme solvant, à une température comprise entre -80 et +80°C, ou
   b) on fait réagir du 2-formyldihydropyranne avec de l'allyllithium, selon un rapport molaire valant de 1 : 0,8 à 1 : 1,3, en présence d'un éther ou d'un mélange d'éther et d'alcane, comme solvant, à une température comprise entre -80 et +70°C, et
   c) on hydrolyse le produit de réaction obtenu selon a) ou b), et ensuite, pour le cas où $R^2$ est un reste alkyle, on l'éthérifie, d'une manière connue en soi, avec un halogénure de formule $R^2X$ (X = halogène).

3. Procédé selon la revendication 2, caractérisé en ce qu'on effectue la réaction à une température comprise entre 30 et 70°C dans le mode de procédé a), et à une température comprise entre -80 et +20°C dans le

mode de procédé b).

4. Utilisation des composés selon la revendication 1 comme masses de scellement ou agents de revêtement à durcissement cationique pour des supports plats ou comme agents réactifs de modification ou de dilution pour des monomères ou des polymères à durcissement cationique.